# EUROPEAN PATENT APPLICATION

(11) **EP 0 970 665 A2**
(43) Date of publication of application: **12.01.2000**
(21) Application number: 99305191.1
(22) Date of filing: 01.07.1999
(51) Int. Cl.: A61F 2/36

(54) **Prosthetic component**

(30) Priority: 01.07.1998 GB 9814267
(71) Applicant: Johnson & Johnson Medical Limited, Ascot, Berkshire SK5 9EY (GB)
(72) Inventor: Griffiths, Brian, Lymington, Hampshire (GB); Naybour, John, Mold, Flintshire (GB); Moore, David, Elmesthorpe, Leicestershire LE9 7SA (GB)
(74) Representative: Fisher, Adrian John

(57) **Abstract**

A prosthetic component which may be in the form of a hip stem (1) is provided with a proximal portion (2) and a distal portion (4) terminating at a distal end (5). The distal portion (4) has at least two slots (6) disposed longitudinally in the distal portion (4). The distal portion (4) of the hip stem (1) has a hollow centre (8). The slots (6) in the distal portion (4) of the hip stem (1) have variable widths along the lengths of the slots (6). The slots (6) can be machined through the wall (9) of the distal portion (4) resulting in a hollow centre (8) of the distal portion (4) with a non-uniform volume.

## Description

This invention relates to a prosthetic component for insertion in a patient's bone. In particular the invention is described in the form of a prosthetic hip stem for insertion in a femur, although the prosthetic component of the invention could be for a shoulder, knee or other application.

In the case of prosthetic hip stems, cementless prosthetic hip stems are known in which a prosthetic hip stem is inserted in a patient's femur without surrounding bone cement. The distal portion of the hip stem gives initial stability within the bone cavity. In the long term, bone growth holds the proximal portion of the hip stem in place. In order to suit different patients, five different sizes of proximal portions of the hip stem are usually available to the surgeon. In addition, four different sizes of distal portions of the hip stem are also available. This results in a range of 20 differently shaped and sized hip stems from which the surgeon can choose the correct size for a particular patient.

If the distal diameter of the hip stem can be modified during an operation, this removes the need for hip stems with different distal portions to be available to the surgeon. The number of hip stems could be reduced to five for the five different proximal portions.

A known form of hip stem with an expandable distal portion has a distal portion with at least two slots of uniform width extending from the distal end of the hip stem. Mandrels are forced into the slots to bend the distal portion of the hip stem outwardly thereby expanding the diameter of the distal portion of the hip stem.

A disadvantage of distal slots of this nature is the full bending stress of each leg is centred at the end of the slots where bending occurs. Up to 2.5mm expansion to the diameter of the distal portion of the hip stem has been found to be possible.

According to a first aspect of the present invention, there is provided a prosthetic component for insertion in a patient's bone comprising a portion of the component which is hollow with at least two longitudinal slots formed therein and wherein the width of the slots varies along the length of the slots.

Preferably, the portion of the component with the slots is a distal portion which is inserted furthest into the patient's bone. Preferably, the component also has a proximal portion without slots formed therein.

The prosthetic component may be a prosthetic hip stem. Alternatively, the prosthetic component may be a shoulder or knee component.

The width of the slots may be curved along the length of the slots. The slots may be of elliptical shape. Alternatively, a first portion of the slots may have a first width and a second portion of the slots may have a second width, wherein the second width is greater than the first width. The second portion of the slots may be distal to the first portion.

Preferably, there are at least four slots in the distal portion. The slots may extend to an end of the component. Alternatively, the slots may terminate before the an end of the component. The distal end of the component may be closed.

The distal portion of the component has a hollow centre with a variable volume along the length of the distal portion. The shape of the hollow centre may conform to the shape of the slots. The hollow centre may be in the form of an ellipsoid.

The areas of the distal portion between the slots form legs with a variable width. The legs may have a triangular cross-section.

According to a second aspect of the present invention, there is provided a method of manufacturing a prosthetic component comprising cutting slots in a portion of the component, the slots varying in width to form a component with a hollow centre of non-uniform volume along the length of the slotted portion of the component.

Preferably, slots are cut in a distal portion of the component which, in use, is inserted furthest into the patient's bone. Preferably, slots are not cut in a proximal portion of the component.

Preferably, opposing slots are cut through the portion of the component.

The end of the component may be solid or, altematively, the slots may extend to an end of the component.

The slots may be cut in an elliptical shape resulting in a stem with a hollow centre of ellipsoid volume.

Embodiments of prosthetic components in accordance with the present invention in the form of prosthetic hip stems are now described, by means of example only, with reference to the accompanying drawings in which:
Figure 1 is a side perspective view of a first embodiment of a distal portion of a prosthetic hip stem in accordance with the present invention;
Figure 2 is a cross-section through the hip stem of Figure 1;
Figure 3 is a partially transparent side view of the hip stem of Figure 1;
Figure 4 is a front elevation of a second embodiment of a hip stem in accordance with the present invention, before expansion;
Figure 5 is a side elevation of the hip stem of Figure 4;
Figure 6 is a front elevation of a third embodiment of a hip stem in accordance with the present invention;
Figures 7a, 7b and 7c are alternative cross-sections of the hip stem of Figure 6;
Figure 8 is a side elevation of the hip stem of Figure 6;
Figures 9a and 9b are a side elevation and a cross-section of a first arrangement of slots in a distal portion of a hip stem in accordance with the present invention;
Figures 10a and 10b are a side elevation and a cross-section of a second arrangement of slots in a distal portion of a hip stem in accordance with the present invention;
Figures 11a, 11b, 11c and 11d are a side elevation, first detail, second detail and cross-section of a third arrangement of slots in a distal portion of a hip stem in accordance with the present invention;
Figures 12a, 12b and 12c are a side elevation, a detail and a cross-section of a fourth arrangement of slots in a distal portion of a hip stem in accordance with the present invention.
Figures 13a, 13b, 13c and 13d are a side elevation, a first detail, a second detail and a cross-section of a fifth arrangement of slots in a distal portion of a hip stem in accordance with the present invention;
Figures 14a, 14b and 14c are a side elevation, a detail and a cross-section of a sixth arrangement of slots in a distal portion of a hip stem in accordance with the present invention; and
Figure 15a is a diagrammatic section of the distal end of a prosthetic component in accordance with the present invention with a slot dividing legs which diverge towards the distal end of the prosthesis and showing the cross-sections along the a leg of the prosthesis adjacent the slot;
Figure 15b is a diagrammatic section of the distal end of a prosthetic component with a slot but with non-diverging legs with the deformation of a leg shown in broken lines; and
Figure 15c is a diagrammatic section as shown in Figure 15b but with an increasing width of slot resulting in a narrowing leg with the deformation of the leg shown in broken lines.

Referring to the drawings, a prosthetic component in the form of a hip stem 1 is provided with a proximal portion 2 with a neck 3 for attachment to a condyle portion. The hip stem 1 has a distal portion 4 which is generally cylindrical terminating at a distal end 5.

A plurality of slots 6 are formed in the distal portion 4 of the hip stem 1. The slots 6 extend longitudinally along the distal portion 4 of the hip stem 1. The slots 6 are equally spaced circumferentially around the cylindrical shape of the distal portion 4. Alternate of the slots 6 may be staggered longitudinally.

The distal portion 4 of the hip stem 1 has a hollow centre 8 surrounded by the wall 9 of the distal portion 4.

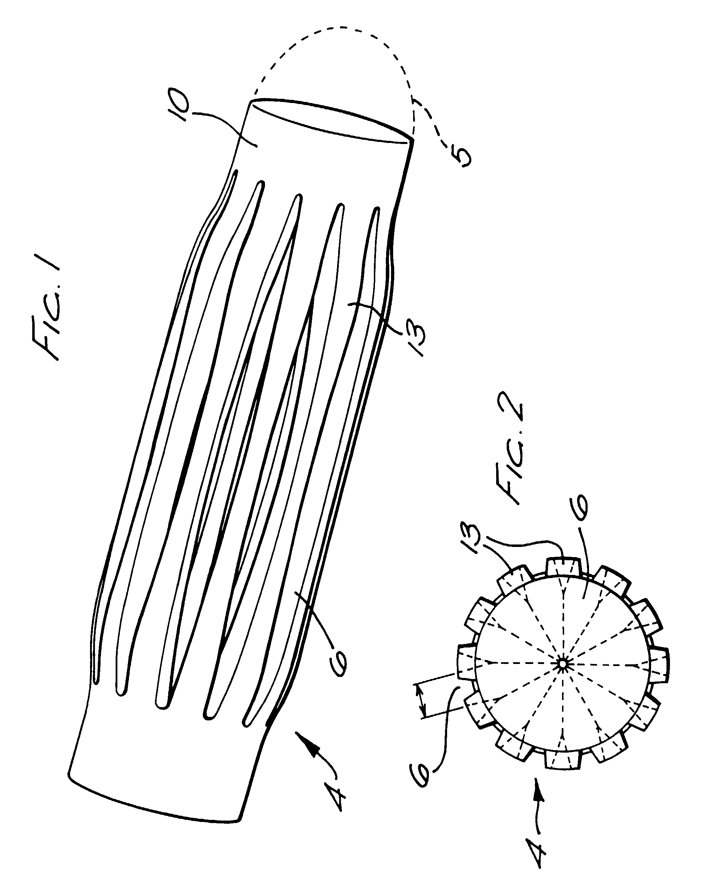

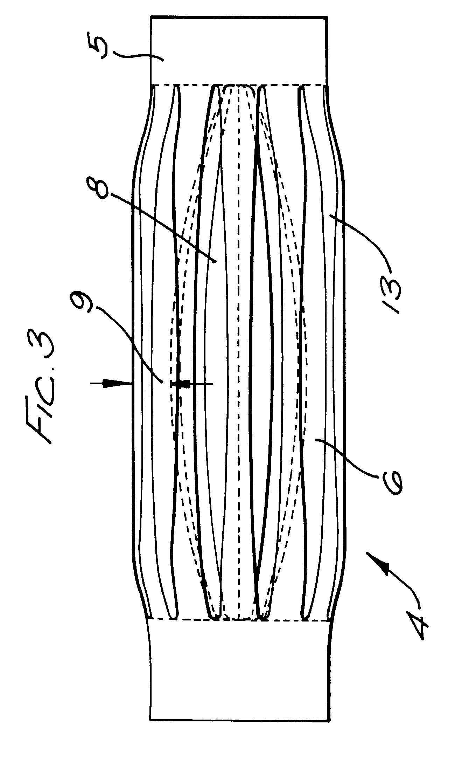

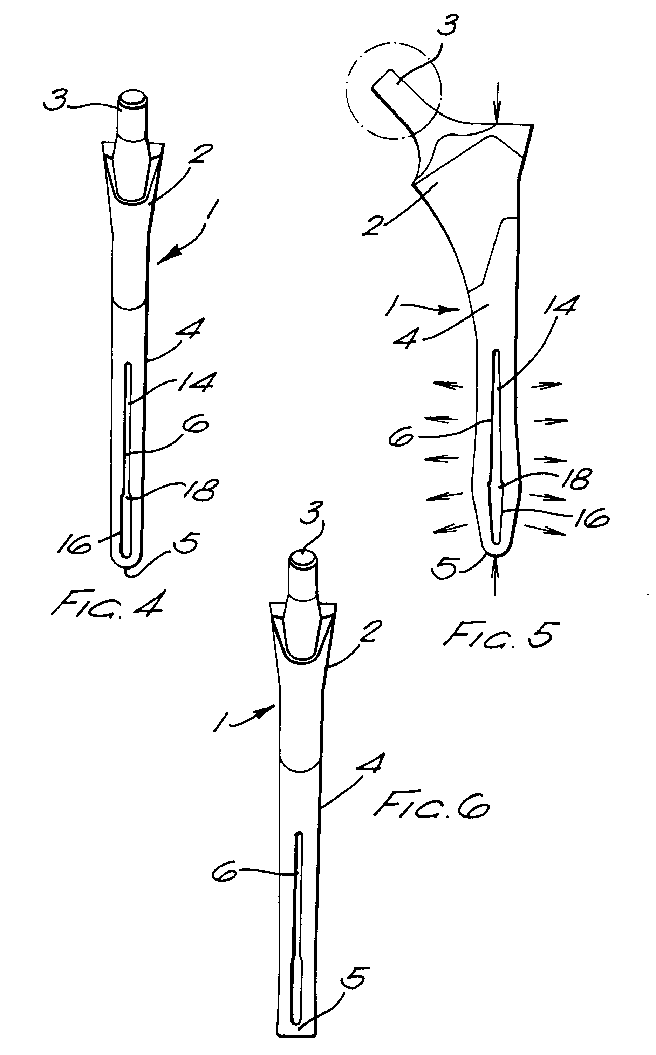

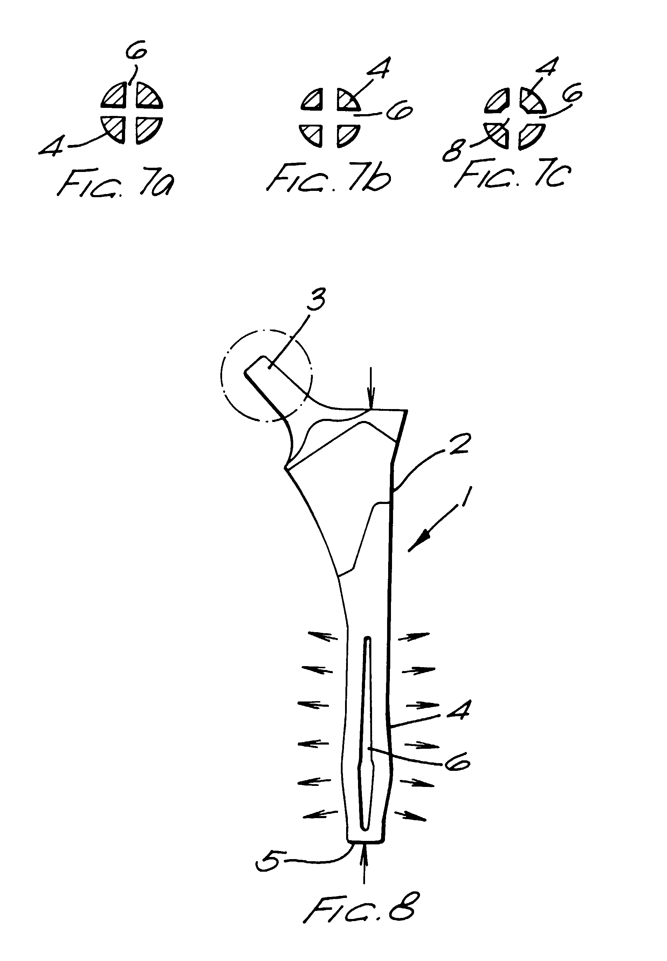

The slots 6 may extend fully to the distal end 5 of the hip stem 1. Altematively, the slots 6 may stop a short distance from the distal end 5 of the hip stem 1 such that a closed ring 10 is provided at the distal end 5. The distal end 5 can be closed as shown in broken lines in Figure 1.

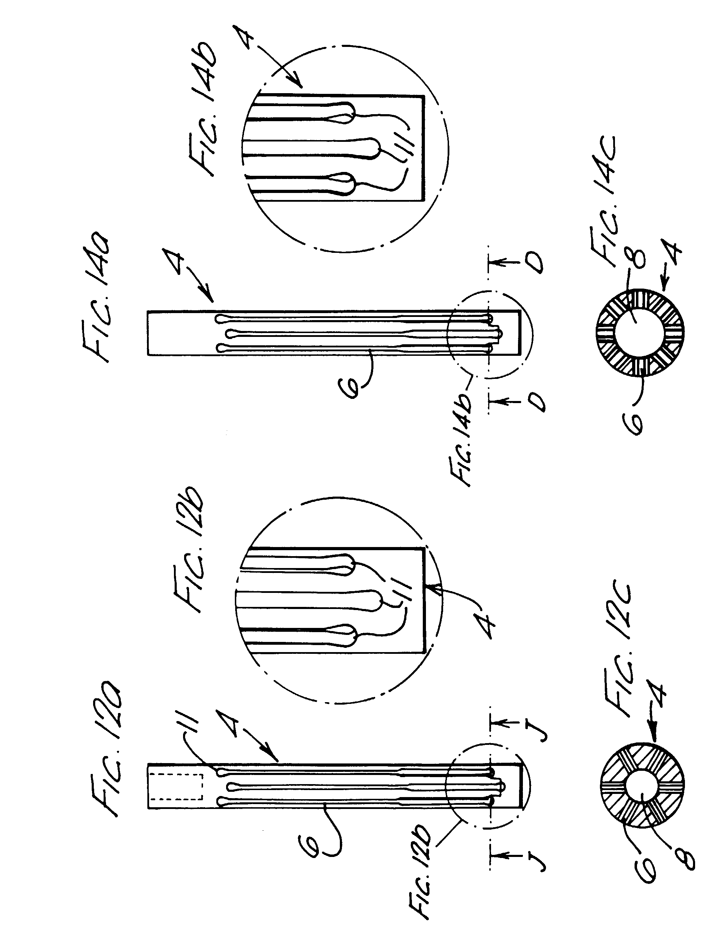

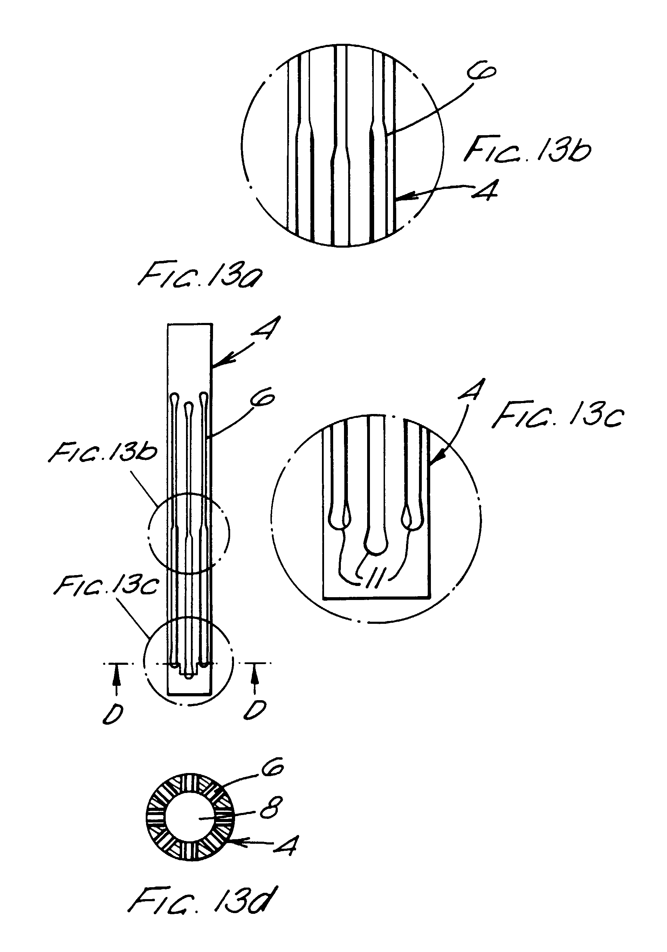

Each slot 6 has a width which varies along the length of the slot 6. In a first embodiment shown in Figures 1 to 3, the slots 6 are of elliptical shape with the greatest width of the slots being midway along the slot 6. In this embodiment, the distal end 5 of the hip stem 1 is in the form of a closed ring 10.

The slots 6 of this embodiment are formed by cutting the slots through the wall 9 of the distal portion 4. The slots 6 are cut through the full diameter of the distal portion 4 of the hip stem 1 to an opposing slot 6 such that the material is removed from the distal portion 4. Cutting the slots 6 in this way results in a hollow centre 8 of the distal portion 4 which has an ellipsoid volume.

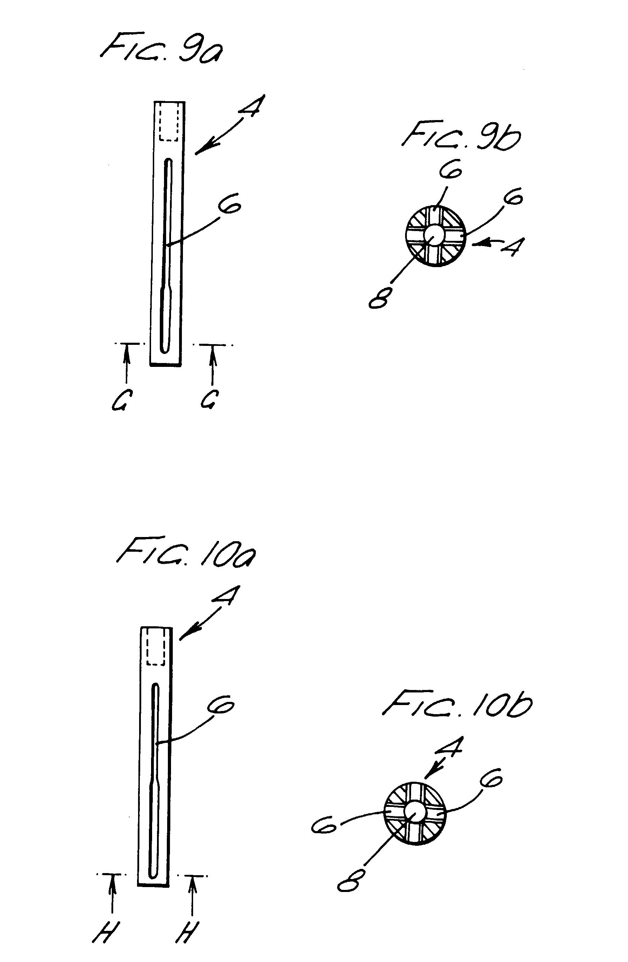

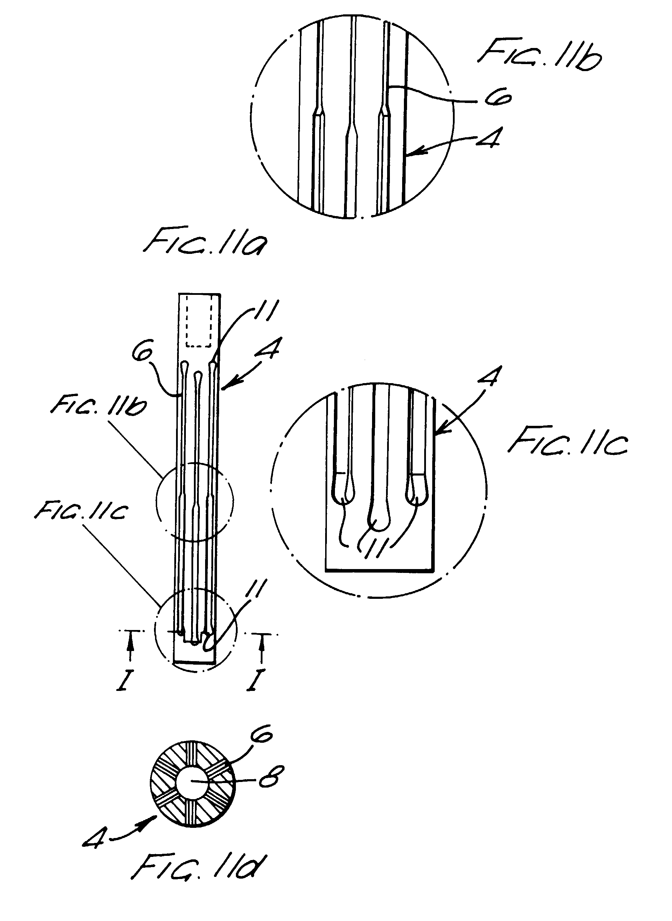

The areas of the wall 9 of the distal portion 4 left between the slots 6 form legs 13. The legs 13 have triangular cross-sections as shown in Figure 2. The ellipsoid shape of the hollow centre 8 results in a variable thickness of wall 9 of the distal portion 4.

In this first embodiment, 10 slots 6 are provided in the distal portion 4 of the hip stem 1.

The hip stem 1 is expanded at its distal portion 4 by applying pressure along the longitudinal length of the hip stem 1. Due to the ellipsoid shape of the hollow centre 8, the distal portion 4 will expand with the greatest expansion occurring at the widest portion of the hollow centre 8. In this way, a controlled expansion of the distal portion 4 can be achieved.

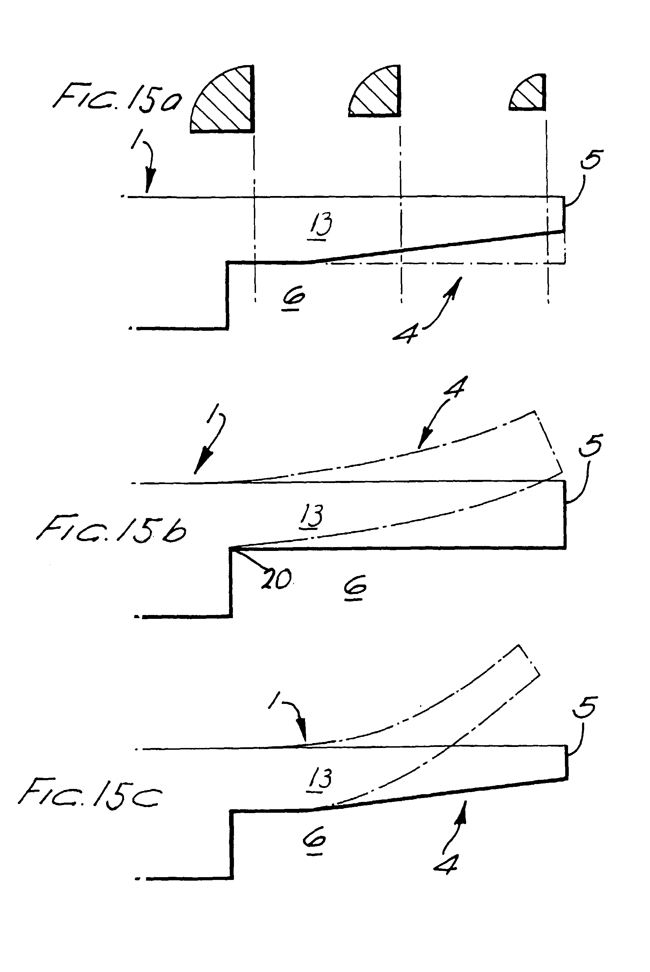

In a second embodiment of the present invention, the slots 6 in the distal portion 4 of the hip stem 1 have a first portion 14 with a first width and a second portion 16 with a second width in which the second width is wider than the first width. The two portions meet at a mid-portion 18 which curves between the two widths. In the embodiment shown in Figures 4 and 5, the distal end 5 of the hip stem 1 is closed and rounded. By varying the width of the slots 6 in this way, the point of maximum expansion moves distally along the hip stem 1 to the second portion 16.

A third embodiment of the hip stem 1 of the present invention is shown in Figures 6 to 8. Various forms of the cross-section of the distal portion 4 are shown.

Figure 9 to 14 show various embodiments of arrangements of slots 6 of varying height and slot density with the first and second portions 14, 16 having first and second widths.

Wider slots 6 have the advantage of removing bone growth into the slots 6 which is advantageous when a hip stem 1 is removed for revision surgery.

Although the embodiments illustrated above have closed ends at the distal end 5 of the hip stem 1, the variable width of slot can be used with hip stems 1 in which the slots 6 extend to the end of the hip stem 1. This has the advantage of reducing the stress of bending at the end of the slots 6 as the legs 13 curve outwardly. The prosthetic component can be manufactured by separate preparation of the legs and subsequent attachment to the prosthesis.

Figures 15a and 15c show a prosthetic component, which may be a hip stem, with slots 6 which are open at their distal ends. The increase in width of the slots 6 along their length reduces the stiffness along the length of the legsl3 between the slots 6 which have correspondingly reduced widths. Figure 15a shows cross-sections of a leg 13 along its length.

Figure 15b is a comparative example of the deformation of a leg 13 of constant width. The deformed leg 13 is shown in broken lines. All the bending takes place at the point of greatest stress at the junction 20 of the leg 13 and the end of the slot 6. The corresponding Figure 15c of a component in accordance with the present invention shows the deformation of a leg 13 of reduced width. By reducing the section stiffness along the length, the stress can be made the same along the length of the leg 13 and therefore the deflection for a given load will be greater.

The prosthetic component and method of manufacture have been described by means of an example of a hip stem. The prosthetic component of the present invention can be used in shoulder, knee and other applications with the expandable portion being at any desired portion of the component.

Modifications and improvements can be made to the foregoing without departing from the scope of the present invention.

## Claims

1. A prosthetic component for insertion in a patient's bone comprising a portion of the component which is hollow and with at least two longitudinal slots formed therein and wherein the width of the slots varies along the length of the slots.

2. A prosthetic component as claimed in claim 1, wherein the portion of the component with the slots is a distal portion which in inserted furthest into the patient's bone.

3. A prosthetic component as claimed in claim 2, wherein the component has a proximal portion without slots formed therein.

4. A prosthetic component as claimed in claim 1 or claim 2, wherein the component is a hip stem.

5. A prosthetic component as claimed in claim 1 or claim 2, wherein the component is a shoulder or knee component.

6. A prosthetic component as claimed in any of the preceding claims, wherein the widths of the slots have edges which are curved along the length of the slots.

7. A prosthetic component as claimed in any of the preceding claims, wherein the slots are of elliptical shape.

8. A prosthetic component as claimed in any of claims 1 to 5, wherein a first portion of the slots have a first width and a second portion of the slots have a second width, wherein the second width is greater than the first width.

9. A prosthetic component as claimed in claim 8, wherein the second portion of the slots is distal to the first portion.

10. A prosthetic component as claimed in any of the preceding claims, wherein there are at least four slots in the distal portion.

11. A prosthetic component as claimed in any of the preceding claims, wherein the slots extend to an end of the component.

12. A prosthetic component as claimed in any of claims 1 to 10, wherein the slots terminate before an end of the component.

13. A prosthetic component as claimed in claim 12, wherein the distal end of the component is closed.

14. A prosthetic component as claimed in any of the preceding claims, wherein the distal portion of the component has a hollow centre with a variable volume along the length of the slotted portion.

15. A prosthetic component as claimed in claim 14, wherein the shape of the hollow centre conforms to the shape of the slots.

16. A prosthetic component as claimed in claim 14 or claim 15, wherein the hollow centre is in the form of an ellipsoid.

17. A prosthetic component as claimed in any of the preceding claims, wherein the areas of the slotted portion between the slots form legs with a variable width.

18. A prosthetic component as claimed in claim 17, wherein the legs have a cross-section in the form of sectors of a circle or of an annulus.

19. A method of manufacturing a prosthetic component comprising cutting slots in a portion of the component, the slots varying in width to form a component with a hollow centre of non-uniform volume along the length of the slotted portion of the component.

20. A method of manufacturing a prosthetic component as claimed in claim 19, wherein slots are cut in a distal portion of the component which, in use, is inserted furthest into the patient's bone.

21. A method of manufacturing a prosthetic component as claimed in claim 19 or claim 20, wherein slots are not cut in a proximal portion of the component.

22. A method of manufacturing a prosthetic component as claimed in any of claims 19 to 21, wherein opposing slots are cut through the distal portion of the stem.

23. A method of manufacturing a prosthetic component as claimed in any of claims 19 to 22, wherein the distal end of the stem is solid.

24. A method of manufacturing a prosthetic component as claimed in any of claims 19 to 22, wherein the slots extend to the distal end of the stem.

25. A method of manufacturing a prosthetic stem as claimed in any of claims 19 to 24, wherein the slots are cut in an elliptical shape resulting in a stem with a hollow centre of ellipsoid volume.
